(19) Europäisches Patentamt
European Patent Office
Office européen des brevets

(11) Veröffentlichungsnummer: **0 004 024**
**B1**

(12) # EUROPÄISCHE PATENTSCHRIFT

(45) Veröffentlichungstag der Patentschrift: **24.02.82**

(21) Anmeldenummer: **79100587.9**

(22) Anmeldetag: **28.02.79**

(51) Int. Cl.³: **C 07 D 245/06**
//C07D303/36, C07D209/48

(54) **Verfahren zur Herstellung von 3-Hydroxy-6-phenyl-1,2,3,4-tetrahydro-1,5-benzodiazocinen.**

(30) Priorität: **10.03.78 DE 2810349**

(43) Veröffentlichungstag der Anmeldung:
**19.09.79 Patentblatt 79/19**

(45) Bekanntmachung des Hinweises auf die Patenterteilung:
**24.02.82 Patentblatt 82/8**

(84) Benannte Vertragsstaaten:
**CH DE IT SE**

(56) Entgegenhaltungen:
**keine**
**DE - A - 1 920 908**
**DE - A - 2 221 558**
**DE - A - 2 640 599**

**ACTA PHARM. JUGOSLAV. 26 (1976). Kajfeč.S. 201, 205.**

**CHEMICAL ABSTRACTS, Vol. 87, Nr. 5, August 1., 1977, Columbus, Ohio, USA,**

(73) Patentinhaber: **Kali-Chemie Pharma GmbH**
**Hans-Böckler-Allee 20 Postfach 220**
**D-3000 Hannover 1 (DE)**

(72) Erfinder: **Liepmann, Hans, Dipl.-Chem. Dr.rer.nat.**
**Auf dem Emmerberge 17**
**D-3000 Hannover (DE)**
Erfinder: **Hüschens, Rolf, Dipl.-Chem. Dr.rer.nat.**
**Matthäikirchstrasse 25**
**D-3000 Hannover 81 (DE)**
Erfinder: **Milkowski, Wolfgang, Dipl.-Chem. Dr.rer.nat.**
**Fasanenweg 13**
**D-3167 Burgdorf (DE)**
Erfinder: **Zeugner, Horst, Dipl.-Chem. Dr.rer.nat.**
**Havelweg 10**
**D-3000 Hannover 73 (DE)**

(74) Vertreter: **Lauer, Dieter, Dr. et al,**
**c/o Kali-Chemie Aktiengesellschaft Postfach 220**
**D-3000 Hannover 1 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:

H. LIEPMANN et al.: "Cyclization of $N_1$-aryl-$N_2$-aroyl-2-hydroxy-1,3-diaminopropánes into benzodiazepines and benzodiazocines" Zusammenfassung Nr. 39 446w.

CHEMIE DER HETEROCYCLEN, Berlin 1968, Katritzky et al. S. 144.

JOURNAL OF ORGANIC CHEMISTRY, Vol. 34, Nr. 1, Januar 1969, M. E. DERIEG et al.: "1,5-Benzodiazocines".

JOURNAL OF PHARMACEUTICAL SCIENCES, Vol. 58, Nr. 7 Juli 1969, American Pharmaceutical Ass. MARTIN STEINMAN et al.: "Synthesis and pharmacological properties of some substituted 1,5-Benzodiazocin-2-ones".

PRINCIPLES OF MODERN HETEROCYCLIC CHEMISTRY, New-York 1968. L. A. Paquette, S. 36—37.

Verfahren zur Herstellung von 3-Hydroxy-6-phenyl-1,2,3,4-tetrahydro-1,5-benzodiazocinen

Gegenstand der Erfindung ist ein Verfahren zur Herstellung von 3-Hydroxy-6-phenyl-1,2,3,4-tetrahydro-1,5-benzodiazocinen der allgemeinen Formel I

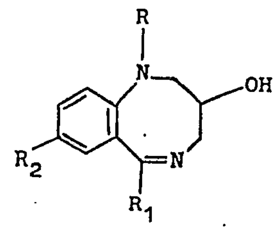

sowie deren Säureadditionssalze, in welcher R ein Wasserstoffatom oder eine Methylgruppe, $R_1$ eine Phenyl-, 2-Halogenphenyl- oder 2-Trifluormethylphenylgruppe und $R_2$ ein Wasserstoffatom, ein Halogenatom, eine Nitrogruppe oder eine Trifluormethylgruppe bedeuten, dadurch gekennzeichnet, daß man 2-Amino-benzophenone der allgemeinen Formel II

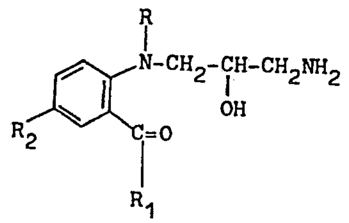

in welcher R, $R_1$ und $R_2$ die obige Bedeutung haben, oder deren Säureadditionssalze bei erhöhter Temperatur in Gegenwart eines inerten organischen Lösungsmittels cyclisiert.

Als inerte organische Lösungsmittel lassen sich bei dieser Cyclisierungsreaktion niedermolekulare ein- oder zweiwertige Alkanole (z.B. Methanol, Äthanol, Propanol, Isopropanol, Butanole oder Äthylenglycol), Essigsäure oder aprotische Lösungsmittel, wie z.B. Benzol, Toluol, Xylol, Dioxan, Tetrahydrofuran, Sulfolan oder Dimethylsulfoxid, einsetzen. Die Cyclisierungsreaktion läßt sich bei Temperaturen zwischen 40 und 200°C durchführen, wobei im allgemeinen Temperaturen zwischen 80 und 160°C bei Normaldruck oder erhöhtem Druck zu guten Ausbeuten führen. Bei Einsatz von Essigsäure werden Temperaturen zwischen 40 und 100°C verwendet.

In manchen Fällen, z.B. bei Einsatz des 2-Aminobenzophenons der allgemeinen Formel II als Base, kann die Cyclisierung durch Zusatz von Ammoniumsalzen, wie Ammoniumchlorid, Ammoniumsulfat, oder Ammoniakgas begünstigt werden.

Die Cyclisierungsbedingungen sind u.a. von den Substituenten des 2-Aminobenzophenons abhängig. Im allgemeinen erfolgt der Ringschluß schneller und vollständiger, wenn das $N_1$-Stickstoffatom methyliert ist. Vor allem bei Substitution des 2-Aminobenzophenons in 5-Stellung kann es vorteilhaft sein, wenn man ein Säureadditionssalz desselben einsetzt, wobei Salze der Salzsäure, Schwefelsäure oder Phosphorsäure besonders geeignet sind.

Nach beendeter Reaktion kann man die Reaktionsprodukte nach üblichen Methoden aufarbeiten und die Produkte als Basen oder in Form ihrer Salze mit anorganischen oder organischen Säuren (Hydrochlorid, Sulfat, Maleinat usw.) isolieren.

Halogen hat in den obigen Formeln in der 2-Halogenphenylgruppe $R_1$ die Bedeutung Fluor, Chlor oder Brom, in $R_2$ Chlor, Brom oder Jod, vorzugsweise Chlor und Brom.

3-Hydroxy-6-phenyl-1,2,3,4-tetrahydro-1,5-benzodiazocine der allgemeinen Formel I sind als Zwischenprodukte für die Herstellung von pharmakologisch wertvollen 1,4-Benzodiazepinderivaten bekannt. Außerdem zeigen 3-Hydroxy-1,5-benzodiazocine eine wertvolle Beeinflussung des Zentralnervensystems. Diese Verbindungen sowie ihre pharmazeutisch verwendbaren Säureadditionssalze sind nützliche Antikonvulsiva, Muskelrelaxantien und Sedativa, wie der DE—OS 23 53 165 zu entnehmen ist.

Aus der DE—OS 22 21 558 ist bereits ein Verfahren zur Herstellung von solchen benzodiazocinen der allgemeinen Formel I bekannt. Dabei werden Acyldiamine der allgemeinen Formel

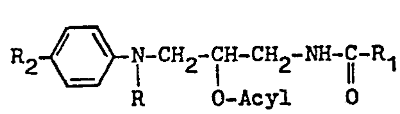

in welcher R, $R_1$ und $R_2$ die obige Bedeutung haben und Acyl vorzugsweise eine Acetyl- oder Benzoylgruppe bedeutet, mit Phosphoroxidchlorid bei Temperaturen zwischen 50 und 150°C, insbesondere 110—130°C, cyclisiert und die hierbei entstehenden 3-Acyloxy-1,5-benzodiazocine in an sich bekannter Weise zu den 3-Hydroxy-1,5-benzodiazocinen hydrolysiert. Nachteilig bei diesem Verfahren ist, daß die Cyclisierungsreaktion und die anschließende Hydrolyse in Abhängigkeit von den Substituenten an den Phenylringen der Acyldiamine unterschiedlich verläuft und so in vielen Fällen nur unbefriedigende Ausbeuten erzielt werden können.

Es wurde schon vorgeschlagen, durch Cyclisierung von-2-(3-Aminopropylamino)-benzophenonderivaten die 6-Phenyl-1,5-benzodiazocinderivate herzustellen. Dieses Verfahren führt aber nur im begrenzten Umfang zum gewünschten Erfolg. So beschreiben M. E. Derieg, R. M. Schweininger und R. I. Fryer in J. Org. Chem. *34* (1969), S.179—183, daß man ausgehend von 2 - (β - Benzyloxycarbonylalanyl - N - methylamido) - 5 - chlorbenzophenon zu 8 - Chlor - 3,4 - dihydro - 1 - methyl - 6 - phenyl - 1,5 - benzodiazocin - 2(1H) - on kommen kann; der Versuch, die am $N_1$-Stickstoffatom nicht methylierte Verbindung zu cyclisieren, führt jedoch unter Dimerisation zum Tetraazacyclohexadecan-Derivat.

Mit etwas besserem Erfolg können 2-(3-Aminopropylamino)-5-chlorbenzophenone zu den gewünschten 1,5-Benzodiazocinen cyclisiert werden. Bei diesem Verfahren sind die Ausgangsverbindungen aber nur in unvorteilhafter Weise zugänglich. Man erhält sie durch Hylyse von 9 - Chlor - 1,2,3,5 - Tetrahydro - 7 - phenylpyrimido[1,2 - a][1,4] benzodiazepin. Ein anderer Weg führt über die entsprechenden 2 - (3 - Halogenpropylamino) - 5 - chlorbenzophenone. Diese sind aber nicht durch direkte Alkylierung der 2-Aminobenzophenone zugänglich, sondern werden entweder durch Hydrolyse von 7 - Chlor - 1 - (3 - chlorpropyl) - 1,3 - dihydro - 5 - phenyl - 2H - 1,4 - benzodiazepin - 2 - on oder über die reaktive 2 - Tosylamido - 5 - chlorbenzophenon - Zwischenstufe gewonnen.

Die Ausführungen von M. Steinman und J. G. Topliss in J. Pharm. Sci. (1969), S.830—832, bestätigen die oben beschriebene Möglichkeit der Herstellung des 1 - Methyl - 1,5 - Benzodiazocin - 2 - ons über 2 - (β - Benzyloxycarbonylalanyl - N - methylamido) - 5 - chlorbenzophenon. Gleichzeitig zeigen die Autoren aber auf, daß bei Verwendung einer Phthalimidogruppe zum Zwecke der Einführung des Stickstoffs in 5-Stellung nicht das gewünschte 1,5-benzodiazocin-Derivat erhalten wird, sondern daß sich nur ringverengte Quinolone bilden.

Demgegenüber verläuft die erfindungsgemäße Cyclisierung der für die Umsetzung erforderlichen 2-(3-Amino-2-hydroxypropylamino)-benzophenone der allgemeinen Formel II trotz der β-ständigen Hydroxylgruppe in glatter Reaktion mit guten Ausbeuten. Dieses ist aus mehreren Gründen überraschend. So war aufgrund von Literaturhinweisen eine Reaktion zu Aminalen unter Ausbildung eines bicyclischen 4,1-Oxepin-1,5-diazocinsystems zu rechnen. Zum andern war nicht auszuschließen, daß bei der Umsetzung der 2-(3-Amino-2-hydroxypropylamino)-benzophenone aufgrund der favorisierten Bildungstendenz eines 7-Ringsystems gegenüber dem 8-Ringsystem nur die 2-(2-Hydroxypropylamino)-gruppe ohne Beteiligung der endständigen 3-Aminogruppe zur 4,1-Oxepin-halbketalstufe reagiert, so daß die Umsetzung zu den angestrebten 3-Hydroxy-1,5-benzodiazocinverbindungen erheblich gestört wird.

Als vorteilhaft erweist es sich ferner, daß die 2-(3-Amino-2-hydroxypropylamino)-benzophenone der allgemeinen Formel II beispielsweise sowohl aus den entsprechenden 2-(3-Halogen-2-hydroxypropylamino)-benzophenonender allgemeinen Formel III direkt mit Ammoniak als auch über die gut zu reinigenden Phthalimid-Verbindungen der allgemeinen Formel IV darstellbar sind. Dabei ist es nicht unbedingt erforderlich, daß man die 2-(3-Amino-2-hydroxypropylamino)-benzophenone der allgemeinen Formel II als Zwischenstufe isoliert, man kann die Cyclisierung zum 3-Hydroxy-1,5-benzodiazocin der allgemeinen Formel I auch in der Reaktionsmischung durchführen. Ein weiterer, nicht vorhersehbarer Vorteil besteht darin, daß man die Verbindungen der allgeminen Formeln III und IV direkt durch Umsetzung der 2-Aminobenzophenone der allgemeinen Formel V mit Epichlorhydrin bzw. N-(2,3-Epoxypropyl)-phthalimid herstellen kann.

Die 2-Aminobenzophenone der allgemeinen Formel II können nach verschiedenen, an sich bekannten Methoden hergestellt werden.

So können beispielsweise Verbindungen der allgemeinen Formel III

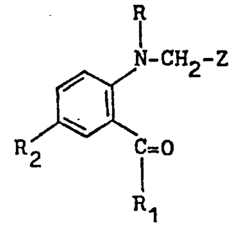

in welcher R, $R_1$ und $R_2$ die obige Bedeutung haber und

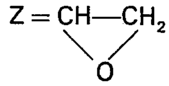

oder —CH(OH)—$CH_2$-Halogen bedeutet, wobei Halogen vorzugsweise Chlor oder Brom ist, mit Ammoniakgas in Gegenwart eines inerten Lösungsmittels umgesetzt werden. Als inerte Lösungsmittel eignen sich z.B. niedermolekulare Alkanole, beispielsweise Methanol, Äthanol, Propanol, Isopropanol oder Butanole, oder Dioxan sowie Tetrahydrofuran. Reaktionstemperaturen zwischen 10 und 60°C führen im allgemeinen zu den gewünschten Verbindungen. Die so erhaltenen Verbindungen der allgemeinen Formel II

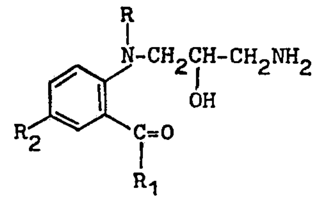

können, wie oben beschrieben, unter Erhitzen und gegebenenfalls unter Druckanwendung cyclisiert werden.

Die Verbindungen der allgemeinen Formel II müssen nicht aus dem Reaktionsmedium, in welchem sie hergestellt wurden, isoliert werden, sondern sie können direkt in die entsprechenden Verbindungen der Formel I durch Erhitzen des Reaktionsgemisches übergeführt werden.

Nach einer weiteren Verfahrensvariante können 2-Aminobenzophenone der allgemeinen Formel II durch saure oder alkalische Spaltung von Verbindungen der allgemeinen Formel IV.

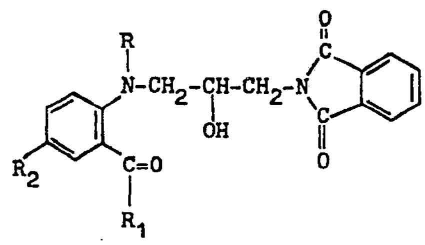

in welcher R, $R_1$ und $R_2$ die obige Bedeutung haben, unter Abspaltung der Phthaloylgruppe erhalten werden. Zur Durchführung der sauren Spaltung werden die Verbindungen der allgemeinen Formel IV vorzugsweise mit konzentrierter wäßriger Salzsäure unter Rückfluß erhitzt. Die Verbindungen der allgemeinen Formel II können aus dem Reaktionsgemisch als Basen oder Hydrochloride isoliert und gereinigt werden. Vorteilhafterweise gewinnt man die Verbindungen der allgemeinen Formel II mit R = Methyl als Hydrochloride. Zur Cyclisationsreaktion können aber auch die Rohöle herangezogen werden.

Bei der alkalischen Abspaltung der Phthaloylgruppe werden die Verbindungen der allgemeinen Formel IV durch Umsetzung mit Hydrazin, Hydrazinhydrat, wasserlöslichen niedermolekularen primären Aminen, wie Methyl-, Äthyl, Propyl- oder Butylamin, vorzugsweise Methylamin, bzw. Aminoäthanol erhalten. Die Umsetzung erfolgt vorzugsweise in Gegenwart von niedermolekularen Alkanolen, wie Methanol, Äthanol oder Isopropanol oder deren Gemischen, gegebenenfalls unter Zusatz von Wasser, bei der Siedetemperatur des Lösungsmittels. Die so erhaltenen Verbindungen der allgemeinen Formel II können in reiner Form oder als Rohöle, wie oben beschrieben, durch Erhitzen auf Temperaturen bis vorzugsweise 160°C in Gegenwart eines Lösungsmittels in die gewünschten 1,5-Benzodiazocine der allgemeinen Formel I übergeführt werden.

Die durch Hydrazinolyse oder Aminolyse erhaltenen Verbindungen der allgemeinen Formel II müssen nicht aus ihrem Reaktionsmedium isoliert werden, sondern können direkt in die entsprechenden Verbindungen der allgemeinen Formel I durch Erhitzen des Reaktionsgemisches übergeführt werden. In manchen Fällen verläuft die anschließende Cyclisierung der Verbindungen der allgemeinen Formel II zu den gewünschten Verbindungen der allgemeinen Formel I mit einer solchen Reaktionsgeschwindigkeit, daß bei der Temperatur, bei welcher der Phthaloylrest abgespalten wird, gleichzeitig eine teilweise bis vollständige Cyclisierung des 2-Aminobenzophenons II stattfindet.

Die Verbindungen der allgemeinen Formel III

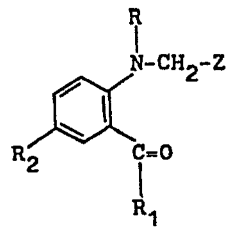

in welcher R, $R_1$, $R_2$ und Z die obigen Bedeutungen haben, lassen sich ebenfalls nach an sich bekannten Methoden gewinnen. Ausgangsverbindungen der allgemeinen Formel III, in welchen Z die Bedeutung —(CH)OH)—$CH_2$-Halogen hat, können durch Umsetzung von 2-Aminobenzophenonen der allgemeinen Formel V

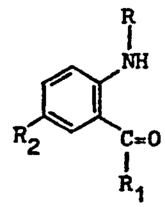

in welcher R, $R_1$ und $R_2$ die obige Bedeutung haben, mit Epichlorhydrin oder Epibromhydrin bei Temperaturen unter 100°C in Gegenwart von beispielsweise Eisessig hergestellt werden.

Ausgangsverbindungen der Formel III, in welchen Z Epoxyäthyl ist, können entsprechend "Houben-Weyl" Methoden der Organischen Chemie 6/3, S. 374 ff. (1965), gewonnen werden, indem die 1,2-Halogenhydrine der Formel III in Gegenwart einer starken Base, wie z.B. Natrium- oder Kaliumhydroxid, und eines inerten Lösungsmittels, wie Äther, Dioxan, Tetrahydrofuran, Benzol oder Toluol bzw. mit konzentrierten wäßrigen Alkalihydroxidlösungen bei Raumtemperatur umgesetzt werden.

Die Phthalimidverbindungen der allgemeinen Formel IV lassen sich aus den Verbindungen der allgemeinen Formel III herstellen, wobei Z beide Bedeutungen haben kann. So können Verbindungen der allgemeinen Formel III, in welchen Z Epoxyäthyl ist, mit Phthalimid bei erhöhter Temperatur in einem inerten Lösungsmittel, z.B. Dimethylformamid, umgesetzt werden. Bei Verwendung der 1,2-Halogenhydrine der allgemeinen Formel III kann man die entsprechenden Phthalimide IV durch Umsetzung der ersteren Verbindungen mit Kaliumphthalimid in Gegenwart eines inerten Lösungsmittels (z.B. Dimethylformamid), durch basenkatalysierte Reaktion, z.B. in Gegenwart von Pyridin, bei erhöhter Temperatur herstellen. Es ist auch möglich, die 2-Aminobenzophenone der allgemeinen Formel V mit N-(2,3-Epoxypropyl)-phthalimid bei erhöhter Temperatur in Gegenwart von Essigsäure zu den Phthalimidoverbindungen der allgemeinen Formel IV umzusetzen.

Zur Herstellung der Verbindungen I, in welchen R eine Methylgruppe ist, kann man von den entsprechenden 2-Methylaminobenzophenonen der allgemeinen Formel V, in welchen $R_1$ und $R_2$ die obige Bedeutung haben, ausgehen. Man kann auch die Methylgruppe in die Verbindungen der allgemeinen Formeln III mit Z = —$CH_2$—CH(OH)—$CH_2$-Halogen oder IV nach aus der Literatur bekannten Methoden einführen, so nach der reduktiven Carbonyl-Aminierung mittels Formalinlösung in Gegenwart von Ameisensäure bei erhöhter Temperatur (s. Leuckart-Wallach-bzw. Eschweiler-Clarke- Reaktion in H. Krauch, W. Kunz, Reaktionen der Organischen Chemie (1976) S. 126 und 131), wenn R in diesen Verbindungen ein Wasserstoffatom ist.

Die nachstehenden Beispiele dienen zur Erläuterung der Erfindung.

## Beispiel 1

8-Chlor-3-hydroxy-6-phenyl-1,2,3,4-tetrahydro-1,5-benzodiazocin.

232 g 2-Amino-5-chlorbenzophenon werden mit 102 g Epichlorhydrin und 60 g Essigsäure 6 h auf 60°C erwärmt. Das beim Abkühlen kristallisierende Produkt wird in Wasser verteilt, abgesaugt und anschließend aus 800 ml Aceton umgelöst. Mit dem aus der eingeengten Mutterlauge erhaltenen Kristallisat beträgt die Ausbeute an 2-(3-Chlor-2-hydroxypropylamino)-5-chlor-benzophenon 279 g (86%). Der Schmelzpunkt liegt bei 105—107°C.

16,2 g des vorstehend beschriebenen 2-(3-chlor-2-hydroxypropylamino)-5-chlorbenzophenons werden in 50 ml Isopropanol und 50 ml Dioxan mit 2,2 g Natriumhydroxid und 4,1 ml Wasser 10 h bei Raumtemperatur umgesetzt. Nach Abziehen der organischen Lösungsmittel im Vakuum wird das Rohöl in Chloroform gelöst. Die Chloroformphase wird mit Wasser neutral gewaschen, über Natriumsulfat getrocknet und filtriert. Nach Abziehen des Chloroforms erhält man ein Öl. Durch Umkristallisieren desselben aus Isopropanol/Petroläther werden 8,0 g (55,6%) 2-(2,3-Epoxypropylamino)-5-chlorbenzophenon mit einem Schmelzpunkt von 61—62°C erhalten.

4 g des vorstehend beschriebenen 2-(2,3-Epoxypropylamino)-5-chlorbenzophenons werden in

400 ml Methanol mit 12,0 g Ammoniakgas 20 h bei Raumtemperatur umgesetzt. Nach Abziehen des Lösungsmittels im Vakuum kristallisieren 3,6 g (85%) 2-(3-Amino-2-hydroxypropylamino)-5-chlorbenzophenon aus Isopropanol/Petroläther; die Substanz schmilzt bei 125—129°C.

3,0 g des vorstehend beschriebenen 2-(3-Amino-2-hydroxypropylamino)-5-chlorbenzophenons werden in 100 ml Methanol mit 0,6 g Ammoniumchlorid 20 h im Stahlautoklaven auf 140°C erhitzt. Das Lösungsmittel wird im Vakuum abgezogen, die Substanz aus Chloroform isoliert und durch Chromatographie an Aluminiumoxid der Aktivitätsstufe II mit Chloroform/Methanol gereinigt. Es werden 2,0 g (70,9%) 8 - Chlor - 3 - hydroxy - 6 - phenyl - 1,2,3,4 - tetrahydro - 1,5 - benzodiazocin als Öl erhalten. Das aus Isopropanol/Äther kristallisierte Hydrochlorid schmilzt bei 206—208°C.

Beispiel 2

8-Chlor-1-methyl-3-hydroxy-6-phenyl-1,2,3,4-tetrahydro-1,5-benzodiazocin.

16,2 g 2-(3-Chlor-2-hydroxypropylamino)-5-chlorbenzophenon werden in 19 ml Ameisensäure und 9,5 ml 37%-iger Formalinlösung 2,5 h auf dem Wasserbad erhitzt. Man gießt anschließend die Lösung auf Eis und neutralisiert mit verdünnter wäßriger Natriumhydroxidlösung. Das Reaktionsprodukt wird in Chloroform überführt, die Lösung mit Natriumcarbonatlösung gewaschen, getrocknet und das Chloroform abgezogen. Es werden 19,2 g Rohöl erhalten, das 78% 2-(3-Chlor-2-hydroxypropyl-methylamino)-5-chlorbenzophenon enthält, was einer Ausbeute von 88,6% entspricht. Die aus Isopropanol/Petroläther kristallisierte Substanz schmilzt bei 71—72°C. Das Rohöl kann ohne Reinigung weiter eingesetzt werden.

18,6 g dieses Rohöls werden in 30 ml Isopropanol mit 2,4 g Natriumhydroxid in 4,5 ml Wasser 12 h bei Raumtemperatur umgesetzt. Die filtrierte Lösung wird im Vakuum abgezogen, das Rohöl in Chloroform aufgenommen und aufgearbeitet. Es werden 15 g 2-(2,3-Epoxypropyl-methylamino)-5-chlorbenzophenon als Öl erhalten.

9 g dieses 2-(2,3-Epoxypropyl-methylamino)-5-chlorbenzophenons werden im Stahlautoklaven 10 h bei 150°C mit 5,1 g Ammoniakgas in 100 ml Äthanol umgesetzt. Nach Abziehen des Lösungsmittels im Vakuum wird ein nach HPLC-Analyse 85,1% 8 - Chlor - 1 - methyl - 3 - hydroxy - 6 - phenyl - 1,2,3,4 - tetrahdyro - 1,5 - benzodiazocin enthaltendes Rohprodukt isoliert. Die aus Äther kristallisierte Verbindung hat einen Fp. von 169—170°C.

Beispiel 3

8-Chlor-3-hydroxy-6-phenyl-1,2,3,4-tetrahydro-1,5-benzodiazocin.

64,8 g des aus 2-Amino-5-chlorbenzophenon hergestellten 2-(3-Chlor-3-hydroxypropylamino)-5-chlorbenzophenons, gelöst in 200 ml Dimethylformamid, werden bei 130°C zu einer Suspension, hergestellt aus 40,5 g Kaliumphthalimid, 1,2 ml Pyridin und 60 ml Dimethylformamid, so zugetropft, daß sich die Temperatur bei der exothermen Umsetzung im Bereich von 130—140°C einstellt. Anschließend wird die Lösung weitere 1,5 h bei der Reaktionstemperatur belassen. Nach dem Abziehen des Lösungsmittels im Vakuum wird die Substanz aus Äther isoliert und das Rohöl aus Isopropanol kristallisiert. Es werden 64 g (73,6%) 2-(3-Phthalimido-2-hydroxypropylamino)-5-chlorbenzophenon erhalten; die Substanz schmilzt bei 131—133°C.

22 g des vorstehend beschriebenen 2-(3-Phthalimido-2-hydroxypropylamino)-5-chlorbenzophenons werden in 400 ml Methanol mit 5,5 g Hydrazinhydrat 1,5 h unter Rückfluß erhitzt. Die abgekühlte Lösung wird mit wäßriger Salzsäure angesäuert und filtriert, das Filtrat im Vakuum eingeengt. Die Base wird nach Alkalischstellen mit wäßriger Natriumhydroxidlösung aus Chloroform isoliert. 11,0 g (71,4%) an 2-(3-Amino-2-hydroxypropylamino)-5-chlorbenzophenon kristallisieren aus Isopropanol.

Zur Cyclisierung werden 3,0 g 2-(3-Amino-2-hydroxypropylamino)-5-chlorbenzophenon in 80 ml Dioxan mit 1,7 g Ammoniakgas in 50 ml Methanol 20 h im Autoklaven auf 140°C erhitzt. Nach Aufarbeitung des Reaktionsgemisches werden 1,9 g (67,3%) 8 - Chlor - 3 - hydroxy - 6 - phenyl - 1,2,3,4 - tetrahydro - 1,5 - benzodiazocin erhalten. Der Schmelzpunkt des Hydrochlorids beträgt 206—208°C.

Beispiel 4

8-Chlor-1-methyl-3-hydroxy-6-phenyl-1,2,3,4-tetrahydro-1,5-benzodiazocin.

16,2 g 2-(3-Chlor-2-hydroxypropylamino)-5-chlorbenzophenon werden in 19 ml Ameisensäure und 9,5 ml 37%-iger Formalinlösung 2,5 h auf dem Wasserbad erhitzt. Man gießt anschließend die Lösung auf Eis und neutralisiert mit verdünnter wäßriger Natriumhydroxidlösung. Das Reaktionsprodukt wird in Chloroform überführt, die Lösung mit Natriumbicarbonatlösung gewaschen, getrocknet und das Chloroform abgezogen. Es werden 19,2 g Rohöl erhalten, das 78% 2-(3-Chlor-2-hydroxypropylmethylamino)-5-chlorbenzophenon enthält, was einer Ausbeute von 88,6% entspricht. Die aus Isopropanol/Petroläther kristallisierte Substanz schmilzt bei 71—72°C. Das Rohöl kann ohne Reinigung weiter umgesetzt werden.

18,6 g dieses Rohöls werden in 30 ml Isopropanol mit 2,4 g Natriumhydroxid in 4,5 ml Wasser 12 h bei Raumtemperatur umgesetzt. Die filtrierte Lösung wird im Vakuum abgezogen, das Rohöl in

Chloroform aufgenommen und aufgearbeitet. Es werden 15 g 2-(2,3-Epoxypropyl-methylamino)-5-chlorbenzophenon als Öl erhalten.

15 g des als Rohöl vorliegenden 2-(2,3-Epoxypropyl-methylamino)-5-chlorbenzophenons werden mit 8,1 g Phthalimid und 0,5 ml Pyridin, gelöst in 100 ml Dimethylformamid, 2 h bei 130—140°C umgesetzt. Die nach dem Abziehen des Lösungsmittels aus Chloroform isolierte Substanz wird in Isopropanol gelöst und das Unlösliche abfiltriert. Es werden 15,0 g 2-(3-Phthalimido-2-hydroxypropyl-methylamino)-5-chlorbenzophenon gewonnen, das bei 132—134°C schmilzt. Aus Toluol/Petroläther erhält man ein bei 128—130°C schmelzendes Kristallisat. Beide Kristallsysteme sind ineinander überzuführen.

12,1 g des 2-(3-Phthalimido-2-hydroxypropyl-methylamino)-5-chlorphenons werden in 75 ml Äthanol mit 1,5 g Hydrazinhydrat 1,5 h unter Rückfluß erhitzt. Die Lösung wird heiß filtriert, das Filtrat im Vakuum eingeengt. Die Base wird in Chloroform nach Behandlung mit wäßriger Natriumhydroxidlösung isoliert. Aus Äther werden 6,8 g (84,0%) 8 - Chlor - 1 - methyl - 3 - hydroxy - 6 - phenyl - 1,2,3,4 - tetrahydro - 1,5 - benzodiazocin erhalten. Die Base hat einen Schmelzpunkt von 169—170°C.

Beispiel 5

8-Chlor-1-methyl-3-hydroxy-6-(2′-chlorphenyl)-1,2,3,4-tetrahydro-1,5-benzodiazocin.

31 g 2-(3-Chlor-2-hydroxypropylamino)-2,5-dichlorbenzophenon werden mit 38 ml Ameisensäure und 19 ml 37%-iger Formalinlösung 3 h am Rückfluß erhitzt. Die auf Eis gegossene Lösung wird mit wäßriger Natriumhydroxidlösung neutralisiert und mit Chloroform extrahiert. Nach Abdestillieren des Chloroforms werden 35,5 g Rohöl erhalten, die nach HPLC-Analyse 55,5% 2-(3-Chlor-2-hydroxypropyl-methylamino)-2′,5-dichlorbenzophenon enthalten.

18,6 g dieses Rohöls, gelöst in 100 ml Dimethylformamid, werden bei 125°C zu einer Suspension von 10,2 g Kaliumphthalimid in 1,5 ml Pyridin und 100 ml Dimethylformamid zugetropft und 2,5 h bei dieser Temperatur belassen. Das nach Abziehen des Lösungsmittels aus Chloroform isolierte Rohprodukt wird aus Isopropanol umkristallisiert. Es werden 6,2 g 2 - (3 - Phthalimido - 2 - hydroxypropyl - methylamino) - 2′,5 - dichlorbenzophenon vom Fp. 186—188°C erhalten.

6,0 g dieses Produktes werden in 150 ml Äthanol mit 0,7 g Hydrazinhydrat 3 h unter Rückfluß erhitzt. Aus der heiß filtrierten Lösung wird das Lösungsmittel im Vakuum abgezogen. Nach Aufarbeitung des Rückstandes werden 5 g Rohöl erhalten, die nach HPLC-Analyse neben 2-(3-Amino-2-hydroxypropyl-methylamino)-2′,5-dichlorbenzophenon bereits 30% cyclisiertes Produkt enthalten.

Das Rohöl wird in einem Autoklaven in 100 ml Methanol weitere 2 h auf 150°C erhitzt. Dabei steigt der Anteil an cyclisiertem Produkt auf 75%. Nach Abziehen des Lösungsmittels, Reinigung der Base durch Säulenchromatographie an Aluminiumoxid der Aktivitätsstufe II mit Chloroform/Methanol erhält man 2,8 g (67,2%) 8 - Chlor - 1 -methyl - 3 - hydroxy - 6 - (2′ - chlorphenyl) - 1,2,3,4 - tetrahydro - 1,5 - benzodiazocin. Die aus Äther umkristallisierte Base hat einen Fp. von 176—178°C. Das aus Aceton umkristallisierte Hydrochlorid schmilzt bei 195°C.

Beispiel 6

8-Chlor-1-methyl-3-hydroxy-6-(2′-chlorphenyl)-1,2,3,4-tetrahydro-1,5-benzodiazocin

29,0 g 2-(3-Chlor-2-hydroxypropylamino)-2′,5-dichlorbenzophenon, gelöst in 75 ml Dimethylformamid, werden zu einer Suspension von 16,4 g Kaliumphthalimid in 1,3 ml Pyridin und 75 ml Dimethylformamid bei 120—130°C getropft und 2,5 h bei dieser Temperatur belassen. Das anschließend aus Chloroform isolierte Rohöl enthält nach HPLC-Analyse 67,8%, bezogen auf das Vorprodukt, an 2-(3-Phthalimido-2-hydroxypropylamino)-2′,5-dichlorbenzophenon. Die reine Base hat einen Fp. von 143—145°C.

39,1 g des Rohöls werden mit 32 ml Ameisensäure und 16 ml 37%-iger wäßriger Formalinlösung 3 h am Rückfluß erhitzt. Nach Aufarbeitung und Umkristallisieren aus Isopropanol werden 13,8 g 2 - (3 - Phthalimido - 2 - hydroxypropyl - methylamino) - 2′,5 - dichlorbenzophenon mit Fp. 186—188°C erhalten.

7,5 g des 2 - (3 - Phthalimido - 2 - hydroxypropyl - methylamino) - 2′,5 - dichlorbenzophenons werden in einem Autoklaven mit 150 ml Methanol und 0,9 g Hydrazin 2 h auf 150°C erhitzt. Nach Aufarbeitung werden 3,3 g (63,5%) 8 - Chlor - 1 - methyl - 3 - hydroxy - 6 - (2′ - chlorphenyl) - 1,2,3,4 - tetrahydro - 1,5 - benzodiazocin mit Fp. 176—178°C erhalten.

Beispiel 7

8-Chlor-3-hydroxy-6-phenyl-1,2,3,4-tetrahydro-1,5-benzodiazocin

19,6 g 5-Chlor-2-aminobenzophenon werden mit 20,0 g N-(2,3-Epoxypropyl)-phthalimid in 50 ml Eisessig 14 h auf etwa 90°C erhitzt. Die mit Chloroform verdünnte Lösung wird auf Eis gegossen und mit wäßriger Natriumhydroxidlösung alkalisch gestellt. Das aus Äther isolierte Produkt wird mit Isopropanol umkristallisiert. Es werden 28,5 g (77,5%) an 2-(3-Phthalimido-2-hydroxypropylamino)-5-chlorbenzophenon erhalten.

4,3 g des vorstehend beschriebenen 2-(3-Phthalimido-2-hydroxypropylamino)-5-chlorbenzo-

phenons werden im Autoklaven mit 0,5 g Methylamin in 150 ml Methanol zunächst 2 h auf 60°C und anschließend 15 h auf 150°C erhitzt. Das nach Abziehen des Lösungsmittels im Vakuum erhaltene Rohöl wird chromatographisch an Aluminiumoxid der Aktivitätsstufe II gereinigt. Es werden 0,9 g (31,7%) 8 - Chlor - 3 - hydroxy - 6 - phenyl - 1,2,3,4 - tetrahydro - 1,5 - benzodiazocin erhalten. Der Schmelzpunkt des Hydrochlorids beträgt 206—208°C.

Beispiel 8

8-Chlor-1-methyl-3-hydroxy-6-phenyl-1,2,3,4-tetrahydro-1,5-benzodiazocin

24,5 g 2-Methylamino-5-chlorbenzophenon werden mit 10,2 g Epichlorhydrin und 6,0 g Essigsäure 72 h auf 70°C erhitzt. Nach der Aufarbeitung werden 33,6 g 2-(3-Chlor-2-hydroxypropyl-methylamino)-5-chlorbenzophenon enthaltendes Rohöl isoliert. Das Produkt wird ohne weitere Reinigung umgesetzt.

Hierzu tropft man 33,6 g des Rohöls gelöst in 80 ml Dimethylformamid bei 130°C zu einer Suspension aus 20 g Kaliumphthalimid, 1,5 ml Pyridin und 80 ml Dimethylformamid. Nach etwa 2 h wird das Lösungsmittel abgezogen. Das aus Toluol isolierte 2 - (3 - Phthalimido - 2 - hydroxypropyl - methylamino) - 5 - chlorbenzophenon kristallisiert nach Zusatz von Petroläther. Die Ausbeute beträgt 22,3 g (49,7%), bezogen auf 2-Methylamino-5-chlorbenzophenon.

2,0 g 2—(3-Phthalimido-2-hydroxypropyl-methylamino)-5-chlorbenzophenon werden in 50 ml Methanol mit 6 ml 33%-iger wäßriger Methylaminlösung 45 min unter Rückfluß erhitzt. Zur Aufarbeitung zieht man das Lösungsmittel im Vakuum ab, löst den Rückstand in Chloroform und wäscht mit Wasser. Nach Trocknen und Abziehen des Lösungsmittels wird der ölige Rückstand mit Äther digeriert. Aus der Ätherlösung werden 1,2 g 8 - Chlor - 1 - methyl - 3 - hydroxy - 6 - phenyl - 1,2,3,4 - tetrahydro - 1,5 - benzodiazocin (89,6%) als Rohöl erhalten. Das durch Umkristallisieren gewonnene Produkt hat einen Schmelzpunkt von 169—170°C.

Beispiel 9

8-Chlor-1-methyl-3-hydroxy-6-(2′-chlorphenyl)-1,2,3,4-tetrahydro-1,5-benzodiazocin.

9,3 g 2-Amino-2′,5-dichlorbenzophenon werden mit 7,8 g N-(2,3-Epoxypropyl)-phthalimid in 17,5 ml Essigsäure 17 h auf 90°C erhitzt. Die Lösung wird auf Eis gegossen und mit Chloroform extrahiert. Nach Abdestillieren des Chloroforms erhält man 15,5 g 2-(3-Phthalimido-2-hydroxypropyl-amino)-2′,5-dichlorbenzophenon als Rohöl. Diese werden mit 14 ml Ameisensäure und 7 ml 37%-iger wäßriger Formalinlösung 3 h unter Rückfluß erhitzt. Nach Aufarbeitung des Reaktionsprodukts wird die aus Chloroform isolierte Substanz aus Isopropanol umkristallisiert. Es werden 5,5 g 2 - (3 - Phthalimido - 2 - hydroxypropyl - methylamino) - 2′,5 - dichlorbenzophenon mit Fp. 186—188°C erhalten.

2,0 g 2-(3-Phthalimido-2-hydroxyropylamino)-2′5-dichlorbenzophenon werden in 20 ml Aminoäthanol 2,5 h auf 150°C erhitzt. Nach Abziehen des überschüssigen Aminoäthanols im Vakuum wird die Base aus Chloroform isoliert. Nach Reinigung durch präparative Schichtchromatographie wird 1 g (72,1%) 8 - Chlor - 1 - methyl - 3 - hydroxy - 6 - (2′ - chlorphenyl) - 1,2,3,4 - tetrahydro - 1,5 - benzodiazocin erhalten, das nach Umkristallisieren aus Äther einen Fp. von 176—178°C hat.

Beispiel 10

8-Chlor-1-methyl-3-hydroxy-6-phenyl-1,2,3,4-tetrahydro-1,5-benzodiazocin.

21,7 g 2-(3-Phalimido-2-hydroxypropylamino)-5-chlorbenzophenon werden in 19 ml Ameisensäure und 9,5 ml 37%-iger wäßriger Formalinlösung 2,5 h unter Rückfluß erhitzt. Nach der Aufarbeitung werden aus Isopropanol/Petroläther 17,5 g (78,1%) 2-(3-Phthalimido-2-hydroxypropyl-methylamino)-5-chlorbenzophenon in kristalliner Form erhalten.

22,5 g 2-(3-Phthalimido-2-hydroxypropyl-methylamino)-5-chlorbenzophenon werden mit 100 ml konzentrierter Salzsäure 7 h unter Rückfluß erhitzt. Die erkaltete Lösung wird filtriert, das Filtrat mit Natriumhydroxid alkalisch gestellt und mit Chloroform extrahiert. Das nach Trocknen und Abziehen des Lösungsmittels im Rohöl enthaltene 2-(3-Amino-2-hydroxypropyl-methylamino)-5-chlorbenzophenon wird in 100 ml Eisessig gelöst und 1 h auf 90°C erwärmt. Anschließend wird das Lösungsmittel abgezogen und die Substanz aus Chloroform isoliert. Es werden 7,7 g (51,1%) 8 - Chlor - 1 - methyl - 3 - hydroxy - 6 - phenyl - 1,2,3,4 - tetrahydro - 1,5 - benzodiazocin mit Schmelzpunkt von 169—170°C erhalten.

Die Base cyclisiert in gleicher Weise durch 8-stündiges Erhitzen unter Rückfluß in Isopropanol. Nach HPLC-Analyse beträgt der 1,5-Benzodiazocingehalt 90%.

In entsprechender Weise cyclisiert das Hydrochlorid unter den oben angegebenen Bedingungen in Eisessig bzw. Isopropanol. Der 1,5-Benzodiazocingehalt nach HPLC-Analyse beträgt 80 bzw. 78%.

Beispiel 11

8-Nitro-1-methyl-3-hydroxy-6-phenyl-1,2,3,4-tetrahydro-1,5-benzodiazocin.

8 g 2-(3-Brom-2-hydroxypropyl-methylamino)-5-nitrobenzophenon werden mit einer Suspension aus 5,6 g Kaliumphthalimid, 2,2 g Pyridin und 200 ml Dimethylformamid 3 h auf 130°C erhitzt, die Lösung anschließend in Wasser verteilt, das anfallende Reaktionsprodukt in Chloroform

aufgenommen und isoliert. Durch Umkristallisieren aus Toluol erhält man 5,8 g (62,0%) 2-(3-Phthalimido-2-hydroxypropylmethylamino)-5-nitrobenzophenon vom Schmelzpunkt 183—188°C.

5,8 g dieser Phthaloylverbindung werden in 60 ml konzentrierter Salzsäure 4 h unter Rückfluß erhitzt, die abgekühlte Lösung mit Chloroform extrahiert und die wäßrige Phase im Vakuum zur Trockne abgezogen. Nach azeotroper Destillation mit Toluol im Vakuum wird das erhaltene 2-(3-Amino-2-hydroxypropyl-methylamino)-5-nitrobenzophenon-hydrochlorid in 100 ml Essigsäure 1 h auf 90°C erwärmt. Nach Abdestillieren des Lösungsmittels im Vakuum und Alkalischstellen mit verdünnter wäßriger Natriumhydroxidlösung lassen sich 2,9 g (73,8%) 8 - Nitro - 1 - methyl - 3 - hydroxy - 6 - phenyl - 1,2,3,4 - tetrahydro - 1,5 - benzodiazocin nach Extraktion mit Chloroform isolieren. Die aus Methanol umkristallisierte Substanz besitzt einen Schmelzpunkt von 206—209°C.

Beispiel 12

8-Chlor-3-hydroxy-6-phenyl-1,2,3,4-tetrahydro-1,5-benzodiazocin.

6,4 g 2-(3-Chlor-2-hydroxypropylamino)-5-chlorbenzophenon in 150 ml Methanol werden im Autoklaven mit 10,2 g Ammoniakgas 18 h auf 60°C erhitzt. Nach Abziehen des Lösungsmittels im Vakuum wird die Substanz in Chloroform gelöst. Nach Behandlung mit verdünnter wäßriger Natriumhydroxidlösung wird das Chloroform im Vakuum abgezogen. Nach Umkristallisieren des Rückstandes aus Isopropanol werden 4,4 g (73,1%) 2-(3-Amino-2-hydroxypropylamino)-5-chlorbenzophenon erhalten.

Zur Cyclisierung werden 3 g dieses 2-(3-Amino-2-hydroxypropylamino)-5-chlorbenzophenons in 90 ml Methanol im Autoklaven 18 h auf 90°C erhitzt. Nach Aufarbeitung des Reaktionsproduktes wird 8 - Chlor - 3 - hydroxy - 6 - phenyl - 1,2,3,4 - tetrahydro - 1,5 - benzodiazocin mit 40%-iger Ausbeute erhalten. Der Schmelzpunkt des Hydrochlorids beträgt 206 bis 208°C.

Beispiel 13

8-Chlor-3-hydroxy-6-phenyl-1,2,3,4-tetrahydro-1,5-benzodiazocin.

Im Stahlautoklaven werden 16,1 g 2-(3-Chlor-2-hydroxypropylamino)-5-chlorbenzophenon, gelöst in 170 ml Methanol, mit 8,5 g Ammoniakgas 20 h bei 140°C umgesetzt. Anschließend wird das Lösungsmittel abgezogen, der Rückstand in Chloroform gelöst, die Lösung mit wäßriger Natriumhydroxidlösung und Wasser gewaschen, getrocknet und das Lösungsmittel im Vakuum abgezogen. Das über Aluminiumoxid chromatographisch gereinigte 8 - Chlor - 3 - hydroxy - 6 - phenyl - 1,2,3,4 - tetrahydro - 1,5 - benzodiazocin wird in das Hydrochlorid überführt. Es werden 9,8 g (61,1%) dieser Verbindung mit Fp. 206—208°C erhalten.

Beispiel 14

8-Chlor-1-methyl-3-hydroxy-6-phenyl-1,2,3,4-tetrahydro-1,5-benzodiazocin.

33,8 g 2-(3-Chlor-2-hydroxypropyl-methylamino)-5-chlorbenzophenon werden mit 17,0 g Ammoniakgas in 300 ml Methanol im Stahlautoklaven 12 h auf 150°C erhitzt. Das 8 - Chlor - 1 - methyl - 3 - hydroxy - 6 - phenyl - 1,2,3,4 - tetrahydro - 1,5 - benzodiazocin wird nach Abziehen des Lösungsmittels und Alkalischstellen mit wäßriger verdünnter Natriumhydroxidlösung aus Chloroform isoliert. Die Ausbeute des aus Äther kristallisierenden Produktes beträgt 21,5 g (71,5%). Fp. 169—170°C.

Beispiel 15

8-Chlor-3-hydroxy-6-(2′-chlorphenyl)-1,2,3,4-tetrahydro-1,5-benzodiazocin.

79,8 g 2-Amino-2′,5-dichlorbenzophenon werden mit 30,5 g Epichlorhydrin und 18,2 g Essigsäure 18 h auf 70°C erwärmt. Nach Verdünnen mit Chloroform wird die Lösung mit Wasser neutral gewaschen und anschließend das Lösungsmittel abdestilliert. Es werden 108 g Rohprodukt erhalten, welche nach HPLC-Analyse 75% 2-(3-Chlor-2-hydroxyproyplamino)-2′,5-dichlorbenzophenon enthalten. Das aus Isopropanol/Petroläther kristallisierende Benzophenon hat einen Fp. von 74—76°C.

6,2 g des 2-(3-Chlor-2-hydroxypropylamino)-2′,5-dichlorbenzophenon enthaltenden Rohöls werden in 100 ml Methanol mit 3,4 g Ammoniakgas 18 h bei 150°C im Autoklaven umgesetzt. Das Reaktionsgemisch wird aufgearbeitet und durch Säulenchromatographie gereinigt. Das als Öl anfallende 8 - Chlor - 3 - hydroxy - 6 - (2′ - chlorphenyl) - 1,2,3,4 - tetrahydro - 1,5 - benzodiazocin wird in Ätherlösung mit Salzsäuregas in das Hydrochlorid übergeführt. Das aus Aceton umkristallisierte Produkt hat einen Fp. von 174—178°C.

Beispiel 16

8-Chlor-1-methyl-3-hydroxy-6-(2′-chlorphenyl)-1,2,3,4-tetrahydro-1,5-benzodiazocin.

6,3 g 2-(3-Chlor-2-hydroxypropyl)-methylamino)-2′,5-dichlorbenzophenon werden in 50 ml Äthanol gelöst und mit 4,4 g Ammoniakgas in 80 ml Äthanol 20 h im Autoklaven auf 150°C erhitzt. Die nach Aufarbeiten des Reaktionsgemisches erhaltene Rohbase wird chromatographisch gereinigt. Durch Umkristallisieren aus Äther werden 1,2 g (56,5%) 8 - Chlor - 1 - methyl - 3 - hydroxy - 6 - (2′ - chlorphenyl) - 1,2,3,4 - tetrahydro - 1,5 - benzodiazocin mit Fp. 176—178°C erhalten.

Beispiel 17

8-Brom-1-methyl-3-hydroxy-6-(2′-fluorphenyl)-1,2,3,4-tetrahydro-1,5-benzodiazocin.

34,7 g 2-Amino-5-brom-2′-fluorbenzophenon werden mit 12,0 g Epichlorhydrin und 7,1 g Essigsäure 5 h auf 60°C erwärmt. Das anschließend aus Toluol isolierte 2-(3-Chlor-2-hydroxypropylamino)-5-brom-2′-fluorbenzophenon kristallisiert aus Isopropanol/Petroläther mit Schmelzpunkt 119—121°C. Die Ausbeute beträgt 27,2 g (59,6%).

18,2 g 2-(3-Chlor-2-hydroxypropylamino)-5-brom-2′-fluorbenzophenon werden mit 18 ml Ameisensäure und 9 ml 37%-iger wäßriger Formalinlösung 3 h bei Rückflußtemperatur umgesetzt und aufgearbeitet. Dabei fällt ein Rohöl an, das nach HPLC-Analyse 75% 2-(3-Chlor-2-hydroxypropylmethylamino)-5-brom-2′-fluorbenzophenon enthält.

9,6 g dieses Rohöls werden in 130 ml Äthanol mit 3,4 g Ammoniak 20 h bei 80°C umgesetzt und aufgearbeitet. Nach Reinigung durch Säulenchromatographie an Aluminiumoxid der Aktivitätsstufe II mit Chloroform/Äthanol und Umkristallisieren mit Äther erhält man 3,2 g (49%) 8 - Brom - 1 - methyl - 3 - hydroxy - 6 - (2′ - fluorphenyl) - 1,2,3,4 - tetrahydro - 1,5 - benzodiazocin mit Schmelzpunkt 223°C.

Beispiel 18

3-Hydroxy-6-phenyl-1,2,3,4-tetrahydro-1,5-benzodiazocin.

17,8 g 2-Aminobenzophenon werden mit 9,2 g Epichlorhydrin und 5,4 ml Eisessig 18 h bei 60°C umgesetzt. 27,3 g des nach Aufarbeitung isolierten Rohprodukts enthalten nach HPLC-Analyse 75% 2-(3-Chlor-2-hydroxypropylamino)-benzophenon. Das aus Äther kristallisierende Produkt schmilzt bei 73—75°C.

11,7 g des Rohprodukts werden in 100 ml Methanol mit 8,0 g Ammoniakgas 16 h bei 150°C im Autoklaven umgesetzt. Nach Abziehen des Lösungsmittels wird das Reaktionsprodukt zwischen Toluol und verdünnter wäßriger Salzsäure verteilt. Aus der wäßrigen Phase werden nach Alkalischstellen mit wäßriger Natriumhydroxidlösung mit Methylenchlorid 3,1 g (40,2%) 3-Hydroxy-6-phenyl-1,2,3,4-tetrahydro-1,5-benzodiazocin extrahiert. Die aus Äther kristallisierte Substanz schmilzt bei 149—151°C.

Beispiel 19

1-Methyl-3-hydroxy-6-phenyl-1,2,3,4-tetrahydro-1,5-benzodiazocin.

10,6 g 2-Methylaminobenzophenon werden mit 5,2 g Epichlorhydrin und 3,0 g Eisessig 24 h bei 60°C umgesetzt. 14,4 isoliertes, nach HPLC-Analyse 94%-iges 2-(3-Chlor-2-hydroxypropyl-methylamino)-benzophenon werden ohne weitere Reinigung mit 10,0 g Ammoniakgas in 300 ml Methanol 12 h bei 150°C im Autoklaven umgesetzt. Nach Aufarbeitung werden 7,2 g 1-Methyl-3-hydroxy-6-phenyl-1,2,3,4-tetrahydro-1,5-benzodiazocin isoliert. Das Salz der Maleinsäure kristallisiert mit einem Schmelzpunkt von 135—137°C aus Isopropanol.

Beispiel 20

8-Trifluormethyl-1-methyl-3-hydroxy-6-phenyl-1,2,3,4-tetrahydro-1,5-benzodiazocin.

6,0 g 2 - (3 - Phthalimido - 2 - hydroxypropylmethylamino) - 5 - trifluormethylbenzophenon werden in 60 ml konzentrierter Salzsäure 4 h unter Rückfluß erhitzt, die abgekühlte Lösung mit Chloroform extrahiert und die wäßrige Phase im Vakuum zur Trockne abgezogen. Nach azeotroper Destillation mit Toluol im Vakuum wird das erhaltene 2 - (3 - Amino - 2 - hydroxypropyl - methylamino) - 5 - trifluormethylbenzophenon - hydrochlorid in 100 ml Essigsäure 1 h auf 90°C erwärmt. Nach Abdestillieren des Lösungsmittels im Vakuum und Alkalischstellen mit verdünnter wäßriger Natriumhydroxidlösung läßt sich 8 - Trifluormethyl - 1 - methyl - 3 - hydroxy - 6 - phenyl - 1,2,3,4 - tetrahydro - 1,5 - benzodiazocin isolieren als Öl.

## Patentansprüche

1. Verfahren zur Herstellung von 3-Hydroxy-6-phenyl-1,2,3,4-tetrahydro-1,5-benzodiazocinen der allgemeinen Formel I

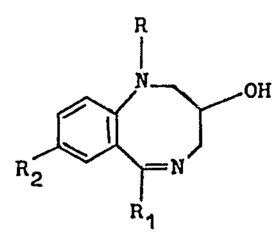

sowie deren Säureadditionssalze, in welcher R ein Wasserstoffatom oder eine Methylgruppe, $R_1$ eine Phenyl-, 2-Halogenphenyl- oder 2-Trifluormethylphenylgruppe und $R_2$ ein Wasserstoffatom, ein Halogenatom, eine Nitrogruppe oder eine Trifluormethylgruppe bedeuten, dadurch gekennzeichnet, daß man 2-Aminobenzophenone der allgemeinen Formel II

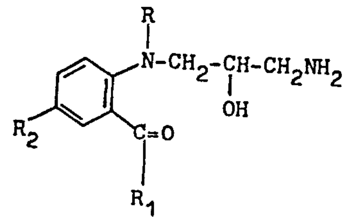

in welcher R, $R_1$ und $R_2$ die obige Bedeutung haben, oder deren Säureadditionssalze bei erhöhter Temperatur in Gegenwart eines inerten organischen Lösungsmittels cyclisiert.

2. Verfahren nach Anspruche 1, dadurch gekennzeichnet, daß man die Cyclisierung bei Temperaturen zwischen 40 und 200°C bei Normaldruck oder erhöhtem Druck durchführt.

3. Verfahren nach den Ansprüchen 1 und 2, dadurch gekennzeichnet, daß man als inerte organische Lösungsmittel niedermolekulare ein- oder zweiwertige Alkohole oder aprotische Lösungsmittel verwendet und die Cyclisierung bei einer Temperatur zwischen 80 und 160°C durchführt.

4. Verfahren nach den Ansprüchen 1 bis 3, dadurch gekennzeichnet, daß man die Cyclisierung der 2-Aminobenzophenone der allgemeinen Formel II im Reaktionsgemisch, in welchem sie hergestellt wurden, durchführt.

5. Verfahren nach den Ansprüchen 1 bis 3, dadurch gekennzeichnet, daß man die Cyclisierung in Gegenwart von Ammoniumsalzen oder Ammoniakgas durchführt.

6. Verfahren nach den Ansprüchen 1 und 2, dadurch gekennzeichnet, daß man als inertes organisches Lösungsmittel Essigsäure verwendet und die Umsetzung bei Temperaturen zwischen 40 und 100°C durchführt.

**Revendications**

1. Procédé pour la production de 3-hydroxy-6-phényl-1,2,3,4-tétrahydro-1,5-benzodiazocines de la formule générale I

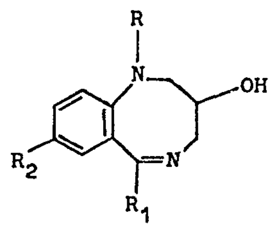

de même que leurs sels d'addition avec les acides, dans laquelle R représente un atome d'hydrogène ou un groupe méthyle, $R_1$ représente un groupe phényle-, 2-halogénophényle ou 2-trifluorométhyle et $R_2$ représente un atome d'hydrogène, un atome d'halogène, un groupe nitro ou un groupe trifluorométhyle, caractérisé en ce qu'on cyclise des 2-aminobenzophénones de la formule générale II

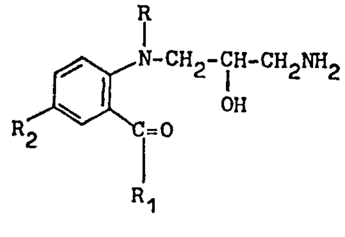

dans laquelle R, $R_1$ et $R_2$ ont les significations indiquées ci-dessus, ou leurs sels d'addition avec les acides, à température élevée en présence d'un solvant organique inerte.

2. Procédé selon la revendication 1 caractérisé en ce qu'on effectue la cyclisation à des températures comprises entre 40 et 200°C à la pression normale ou à une pression plus élevée.

3. Procédé selon la revendication 1 et 2 caractérisé en ce qu'on utilise comme solvant organique inerte, des alcools monovalents ou bivalents de bas poids moléculaire ou un solvant aprotique et qu'on effectue la cyclisation à une température comprise entre 80 et 160°C.

4. Procédé selon les revendications 1 à 3 caractérisé en ce que l'on effectue la cyclisation des 2-aminobenzophénones de la formule générale II dans le mélange réactionnel dans lequel celles-ci ont été préparées.

5. Procédé selon les revendications 1 à 3 caractérisé en ce qu'on effectue la cyclisation en présence de sels d'ammonium ou de gaz ammoniac.

6. Procédé selon les revendications 1 et 2 caractérisé en ce qu'on utilise comme solvants

organiques inertes de l'acide acétique et qu'on effectue la réaction à des températures comprises entre 40 et 100°C.

**Claims**

1. Process for the production of 3-hydroxy-6-phenyl-1,2,3,4-tetrahydro-1,5-benzodiazocines of the general Formula I

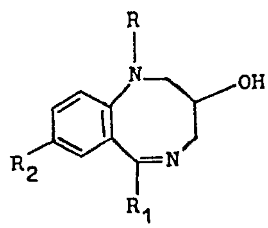

as well as their acid addition salts, in which R is a hydrogen atom or a methyl group, $R_1$ is a phenyl-, 2-halophenyl or 2-trifluoromethyl-phenyl group and $R_2$ is a hydrogen atom, a halogen atom, a nitro group or a trifluoromethyl group, characterised in that 2-aminobenzophenones of the general Formula II

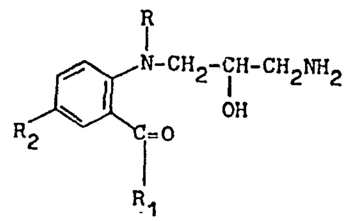

in which R, $R_1$ and $R_2$ have the meanings defined above, or their acid addition salts are cyclised at elevated temperature in the presence of an inert organic solvent.

2. Process according to Claim 1, characterised in that the cyclising is carried out at a temperature between 40 and 200°C under normal pressure or elevated pressure.

3. Process according to Claims 1 and 2, characterised in that the inert organic solvent is a low molecular mono- or dihydric alcohol or an aprotic solvent and the cyclising is carried out at a temperature between 80 and 160°C.

4. Process according to Claims 1 to 3, characterised in that the cyclising of the 2-aminobenzophenones of the general Formula II is carried out in the reaction mixture in which it was produced.

5. Process according to Claims 1 to 3, characterised in that the cyclising is carried out in the presence of ammonium salts or ammonia gas.

6. Process according to Claims 1 and 2, characterised in that the inert organic solvent is acetic acid and the reaction is carried out at a temperature between 40 and 100°C.